# EUROPÄISCHE PATENTSCHRIFT

(11) **EP 4 373 434 B1**
(45) Veröffentlichungstag und Bekanntmachung des Hinweises auf die Patenterteilung: **04.12.2024**
(21) Anmeldenummer: 23771783.0
(22) Anmeldetag: 31.08.2023
(51) Int. Cl.: A61B 50/20, A61B 50/33, A61B 50/34

(54) **BEFESTIGUNGSKLAMMER ZUR ANBRINGUNG VON HALTERN FÜR MEDIZINISCHE INSTRUMENTE IN EINEM STERILISATIONSSIEBKORB UND ANORDNUNG MIT EINEM STERILISATIONSSIEBKORB UND EINER BEFESTIGUNGSKLAMMER**
FASTENING CLAMP FOR ATTACHING HOLDERS FOR MEDICAL INSTRUMENTS IN A STERILISATION MESH BASKET, AND ARRANGEMENT HAVING A STERILISATION MESH BASKET AND A FASTENING CLAMP
PINCE DE FIXATION POUR FIXER DES SUPPORTS POUR INSTRUMENTS MÉDICAUX DANS UN PANIER MAILLÉ DE STÉRILISATION, ET SYSTÈME À PANIER MAILLÉ DE STÉRILISATION ET À PINCE DE FIXATION

(30) Priorität: 02.09.2022 DE 102022122236
(43) Veröffentlichungstag der Anmeldung: 29.05.2024
(73) Patentinhaber: Aesculap AG, 78532 Tuttlingen (DE)
(72) Erfinder: WEIßER, DANIEL, 78048 Villingen-Schwenningen (DE); STREIT, Eva, 78351 Bodman-Ludwigshafen (DE); KNITTEL, TImo, 78573 Wurmlingen (DE); DEUTSCHER, Hermann, 78579 Neuhausen ob Eck (DE)
(74) Vertreter: Winter, Brandl - Partnerschaft mbB
(86) Internationale Anmeldenummer: PCT/EP2023/073892
(87) Internationale Veröffentlichungsnummer: WO 2024/047158

(56) Entgegenhaltungen:
- EP-A1- 2 860 126
- DE-A1- 102005 047 187
- US-A1- 2010 176 016

## Beschreibung

### Technisches Gebiet

Die vorliegende Offenbarung betrifft die Befestigung von Lagerungselementen bzw. Haltern für medizinische Instrumente insbesondere für die minimalinvasive Chirurgie in Sterilisationssiebkörben.

### Hintergrund der Erfindung

In der minimalinvasiven Chirurgie werden medizinische Instrumente in Sterilisationssiebkörben zur Verfügung gestellt und wieder abgelegt. Derartige Sterilisationssiebkörbe mit den Instrumenten werden in verschließbare Sterilisationscontainer eingesetzt und ggf. auch darin gestapelt.

### Stand der Technik

In der EP 2 860 126 A1 und in der US 2010/176016 A1 sind Befestigungsklammern offenbart.

Aus dem Stand der Technik ist ein Sterilisationssiebkorb mit der Bezeichnung Aicon^{®} der Anmelderin bekannt. Er hat einen im Querschnitt betrachtet trapezförmig gestuften Wellenboden, wodurch Füße am Sterilisationssiebkorb entfallen können. Der Wellenboden weist ein Perforationsgitter auf, wobei die Stege um 45 Grad zum Rand des Wellenbodens bzw. zum Rand des Sterilisationssiebkorbs angestellt sind.

Aus dem Stand der Technik ist es weiterhin bekannt, an der Oberseite des Bodens von Sterilisationssiebkörben mittels Befestigungsklammern Instrumentenhalter zu befestigen. Zur Erhöhung der Flexibilität können diese auch versetzt werden. Im Internet unter der Adresse https://endoskopie.bbraun.com/p/PRID00006221 ist eine derartige Befestigungsklammer der Anmelderin mit der Bezeichnung JG301R offenbart. Auch in der DE 10 2005 047187 A1 ist eine derartige Befestigungsklammer offenbart. An einem Hauptabschnitt der Befestigungsklammer sind zwei bezüglich einer Mittelachse einander gegenüberliegenden Armreihen angeordnet, wobei jede Armreihe einen ersten Haltearm und einen zweiten Haltearm hat, an denen jeweils eine seitliche Rastnase gebildet ist, wobei die Rastnasen der beiden Haltearme jeder Armreihe auf einander zu weisen. Die Haltearme der Befestigungsklammer werden von unten durch das Perforationsgitter des Bodens des Sterilisationssiebkorbes gesteckt. Mittels der Rastnasen wird die Befestigungsklammer an dem Perforationsgitter angeclipst. Danach kann ein Silikonsteg des Instrumenthalters zwischen die Haltearme geschoben werden.

Es hat sich an derartigen Befestigungsklammern jedoch gezeigt, dass sie an einem Wellenboden eines fußlosen Sterilisationssiebkorbes (z.B. am zuvor genannten Aicon^{®}-Siebkorb) nicht waagerecht montierbar sind, und dass sie vergleichsweise weit nach unten aus dem Wellenboden herausstehen, so dass der Sterilisationssiebkorb je nach Verteilung der Befestigungsklammern am Wellenboden nicht stabil (z.B. im Sterilisationscontainer) steht. Weiterhin können die nach unten herausstehenden Befestigungsklammern darunter befindliche Gegenstände (z.B. den Boden des Sterilisationscontainers) verkratzen oder sogar beschädigen.

### Kurzbeschreibung der Erfindung

Aufgabe der vorliegenden Offenbarung ist es, eine Befestigungsklammer für einen Wellenboden eines Sterilisationssiebkorbes und eine Anordnung mit einem Sterilisationssiebkorb und mindestens einer derartigen Befestigungsklammer bereitzustellen, bei der der betroffene Sterilisationssiebkorb in allen Konfigurationen bzw. Positionen der Befestigungsklammern stabil steht. Weiterhin soll der darunter befindliche Gegenstand (z.B. den Boden des Sterilisationscontainers) von der Befestigungsklammer nicht verkratzt oder beschädigt werden.

Diese Aufgabe wird in Hinblick auf die Befestigungsklammer mit der Merkmalskombination des Anspruchs 1 gelöst und im Hinblick auf die Anordnung mit der Merkmalskombination des Anspruchs 11 gelöst.

Die Befestigungsklammer gemäß der Offenbarung ist zur werkzeuglosen Befestigung an einem perforierten Wellenboden eines vorzugsweise fußlosen Sterilisationssiebkorbes eingerichtet und ausgelegt. Die Befestigungsklammer hat einen Hauptabschnitt, an dem zwei bezüglich einer Mittelachse einander gegenüberliegenden Armreihen angeordnet sind, wobei jede Armreihe einen ersten Haltearm und einen zweiten Haltearm hat, an denen jeweils eine seitliche Rastnase gebildet ist. Die Rastnasen der beiden Haltearme jeder Armreihe weisen auf einander zu und dienen zum anclipsen der Befestigungsklammer an dem Perforationsgitter des Wellenbodens. An dem Hauptabschnitt der Befestigungsklammer quer zur Mittelachse erstreckt sich zwischen den beiden ersten Haltearmen ein erster Rand und zwischen den beiden zweiten Haltearmen ein zweiter Rand. Erfindungsgemäß ist an den beiden Rändern jeweils eine Aussparung oder Ausnehmung gebildet. Damit ist es möglich, dass der Hauptabschnitt der montierten Befestigungsklammer nicht nach unten aus dem Wellenboden heraussteht, sondern von unten komplett in den Wellenboden eintaucht, weil untere Stegkreuze des Wellenbodens von oben in die Aussparungen des Hautabschnitts eintauchen. Damit kann der Hauptabschnitt darunter befindliche Gegenstände (z.B. einen Boden eines Sterilisationscontainers) nicht verkratzen oder beschädigen.

Vorzugsweise erstrecken sich die beiden Ränder des Hauptabschnitts quer zur Mittelachse.

Bei einer kleineren Version der Befestigungsklammer erstrecken sich die beiden Aussparungen oder Ausnehmungen quer zur Mittelachse vorzugsweise über mindestens ein Drittel einer jeweiligen Breite des Hauptabschnitts oder eines jeweiligen Abstandes der (einander gegenüberliegenden) Haltearme. Damit kann ein Stegkreuz weit in die jeweilige Ausnehmung eintauchen und die Befestigungsklammer besonders hoch montiert werden und/oder der Hauptabschnitt eine vorteilhafte Dicke haben, ohne dass der Hauptabschnitt nach unten aus dem Wellenboden herausragt.

Bei der kleineren Version der Befestigungsklammer erstrecken sich die beiden Aussparungen oder Ausnehmungen entlang der Mittelachse vorzugsweise jeweils über mindestens ein Achtel einer Länge des Hauptabschnitts. Damit kann ein Stegkreuz weit in die jeweilige Ausnehmung eintauchen und die Befestigungsklammer besonders hoch montiert werden und/oder der Hauptabschnitt die vorteilhafte Dicke haben, ohne dass der Hauptabschnitt nach unten aus dem Wellenboden herausragt.

Bei einer größeren Version der Befestigungsklammer ist zwischen den beiden Haltearmen einer Armreihe jeweils ein Zwischenarm vorgesehen. Weiterhin ist etwa mittig im Hauptabschnitt eine weitere Ausnehmung vorgesehen, die als Durchgangsausnehmung ausgebildet ist. Damit können insgesamt drei Stegkreuze in eine jeweilige Ausnehmung eintauchen. Bei der größeren Version der Befestigungsklammer sind vorzugsweise auch an freien Endabschnitten der Zwischenarme Halteabschnitte für den Silikonsteg gebildet.

Vorzugsweise sind an freien Endabschnitten der Haltearme und/oder Zwischenarme Halteabschnitte für einen Silikonsteg eines Instrumentenhalters gebildet. Vorzugsweise sind die Halteabschnitte einander paarweise gegenüberliegend, bevorzugt einander paarweise zugewandt, vorgesehen. Das heißt, bevorzugt weisen die Halteabschnitte der beiden ersten Haltearme auf einander zu und/oder die Halteabschnitte der beiden zweiten Haltearme weisen aufeinander zu und/oder die Halteabschnitte der Zwischenarme weisen aufeinander zu. Bei einer fertigungstechnisch einfachen Ausgestaltung ist die Befestigungsklammer ein Blechbiegeteil. Bei einer fertigungstechnisch und montagetechnisch einfachen Weiterbildung ist die Mittelachse auch eine Symmetrieachse.

Bei einer ersten Variante der größeren Version der Befestigungsklammer ist an den beiden Zwischenarmen jeweils eine weitere Rastnase vorgesehen, die sich entgegen der Rastnase des ersten Haltearms erstreckt. Vorzugsweise ist an den beiden Zwischenarmen jeweils auch eine weitere Rastnase vorgesehen, die sich entgegen der Rastnase des zweiten Haltearms erstreckt. Damit können pro Armreihe zwei Stegkreuze beidseitig gehalten werden, und der Halt der Befestigungsklammer ist verbessert.

Bei einer zweiten Variante der größeren Version ist der Zwischenarm eine Lasche, ohne Rastnasen. Insbesondere ist dazu an der gegenüber den Rastnasen der beiden benachbarten Haltearme jeweils ein gerader Randabschnitt gebildet. Damit werden pro Armreihe ebenfalls zwei Stegkreuze festgeclipst, allerdings nur jeweils einseitig von der Rastnase des Haltearms. Die beiden Randabschnitte jeder Lasche können zueinander parallel sein.

Die Montage der Befestigungsklammer ist vereinfacht, wenn oberhalb der Rastnasen an den vier Haltearmen und im Falle der ersten Variante der größeren Version der Befestigungsklammer auch an den Zwischenarmen jeweils eine Einführschräge gebildet ist. Einander gegenüberliegende Einführschrägen können trichterförmige Freiräume bilden, in die die Stegkreuze eingeführt werden.

Die Anordnung gemäß der Offenbarung hat einen Sterilisationssiebkorb, der zum Einsetzen in einen vorzugsweise fußlosen Sterilisationscontainer eingerichtet und ausgelegt sein kann. Ein Wellenboden des Sterilisationssiebkorbes weist ein Perforationsgitter auf, wobei jeweils vier Stege des Perforationsgitters Stegkreuze bilden. Die Stegkreuze sind auf zwei unterschiedlichen Höhen des Wellenbodens angeordnet. An dem Wellenboden ist mindestens eine vorbeschriebene Befestigungsklammer werkzeuglos anclipsbar oder angeclipst. Dabei kann der Hauptabschnitt der montierten Befestigungsklammer von unten komplett in den Wellenboden eintauchen, weil untere Stegkreuze des Wellenbodens von oben in die Aussparungen des Hauptabschnitts eintauchen. Dann ragt der Hauptabschnitt nicht nach unten aus dem Wellenboden heraus, sondern taucht in diesen ein. Damit kann der Hauptabschnitt darunter befindliche Gegenstände (z.B. den Boden des Sterilisationscontainers) nicht verkratzen oder beschädigen.

Vorzugsweise sind die Stegkreuze in Stegkreuzreihen angeordnet, die abwechselnd auf den beiden unterschiedlichen Höhen angeordnet sind, wobei sich die Stegkreuzreihen entlang dem gesamten Wellenboden und parallel zu einem Rand des Wellenbodens oder des Sterilisationssiebkorbes erstrecken.

Zwischen den beiden Haltearmen jeder Armreihe der mindestens einen Befestigungsklammer ist oder sind jeweils ein oder zwei Stegkreuze einer Stegkreuzreihe aufnehmbar oder aufgenommen. Das eine Stegkreuz oder die beiden Stegkreuze liegen an den Rastnasen an und werden so gehalten. Im Falle der zwei Stegkreuze pro Armreihe erstreckt sich der Zwischenarm der größeren Version der Befestigungsklammer zwischen die beiden Stegkreuze.

Bei der kleineren Version der Befestigungsklammer können zwei zueinander benachbarte Stegkreuze einer unteren Stegkreuzreihe jeweils in eine am Rand gebildete Ausnehmung bzw. Aussparung des Hauptabschnitts eintauchen.

Bei der größeren Version der Befestigungsklammer können drei zueinander benachbarte Stegkreuze einer unteren Stegkreuzreihe jeweils in eine Ausnehmung des Hauptabschnitts eintauchen. Genauer gesagt tauchen zwei Stegkreuze in die beiden am Rand gebildeten Ausnehmungen bzw. Aussparungen ein, während ein Stegkreuz in die mittlere als Durchgangsausnehmung gebildete Ausnehmung eintaucht.

Bei einer besonders bevorzugten Weiterbildung der offenbarten Anordnung sind die Stege des Wellenbodens um 45 Grad zum Rand des Wellenbodens oder des Sterilisationssiebkorbes angestellt. Wegen der Anordnung der Stegkreuze zwischen den Haltearmen einer Armreihe ist trotz der 45 Grad-Anstellung der Stege an der Oberseite des Wellenbodens eine Befestigung der (jeweiligen Mittelachse der) mindestens einen Befestigungsklammer parallel oder unter 90 Grad zum Rand des Wellenbodens oder des Sterilisationssiebkorbes gegeben. Damit erfolgt auch das Einschieben des Silikonstegs des Instrumenthalters zwischen die Haltearme parallel oder unter 90 Grad zum Rand des Wellenbodens oder des Sterilisationssiebkorbes.

Bei der Anstellung der Stege des Wellenbodens um 45 Grad zum Rand des Wellenbodens ist ein besonders tiefes Eintauchen der beiden Stegkreuze in die beiden randnahen Aussparungen bzw. Ausnehmungen möglich und eine möglichst geringe Schwächung des Hauptabschnitts gegeben, wenn die beiden randnahen Aussparungen bzw. Ausnehmungen spiegelsymmetrisch zur Mittelachse sind und jeweils drei etwa rechtwinklig zueinander angestellte Ränder haben. Dann können zwei der vier Stege des betroffenen Stegkreuzes in den beiden Ecken der jeweiligen randnahen Aussparung bzw. Ausnehmung angeordnet werden. Dabei können die beiden Aussparungen bzw. Ausnehmungen auch als Positionierhilfe dienen.

In prinzipiell ähnlicher Weise ist bei der größeren Version ein besonders tiefes Eintauchen des mittleren Stegkreuzes in die als Durchgangsausnehmung ausgebildete weitere Ausnehmung und/oder eine möglichst geringe Schwächung des Hauptabschnitts möglich, wenn die Durchgangsausnehmung rechteckig ist, und wenn die vier Stege des betroffenen Stegkreuzes in den vier Ecken der Durchgangsausnehmung angeordnet werden. Auch bei dieser Ausgestaltung kann die Durchgangsausnehmung als Positionierhilfe dienen.

Der Silikonsteg des Instrumentenhalters wird vorzugsweise unter den Halteabschnitten gehalten, die an den freien Endabschnitten der vier Haltearme und gegebenenfalls auch der beiden Zwischenarme gebildet sind.

Abweichend zum vorteilhaften Eintauchen des Hauptabschnitts von unten in den Wellenboden ist alternativ auch eine untere Montageposition der mindestens einen Befestigungsklammer an dem Wellenboden möglich. Dabei sind zwischen den Haltearmen der beiden Armreihen jeweils Stegkreuze der geringeren Höhe aufgenommen. Auch diese untere Montageposition ist mechanisch definiert und bietet einen sicheren Halt der Befestigungsklammer an dem Wellenboden. Auch bei der unteren Montageposition ist der Hauptabschnitt der Befestigungsklammer eben. Allerdings ragt er dabei nach unten aus dem Wellenboden heraus und dient quasi als Fuß.

### Kurzbeschreibung der Figuren

Fig. 1 ist eine perspektivische Darstellung von oben zur Veranschaulichung einer Anordnung gemäß einem ersten Ausführungsbeispiel der vorliegenden Offenbarung;
Fig. 2 ist eine weitere Darstellung des ersten Ausführungsbeispiels der Anordnung aus Figur 1 ;
Fig. 3 ist eine Darstellung einer Befestigungsklammer gemäß einem zweiten Ausführungsbeispiel der vorliegenden Offenbarung;
Fig. 4 ist eine seitliche Darstellung einer Anordnung mit dem zweiten Ausführungsbeispiel der Befestigungsklammer aus Fig. 3;
Fig. 5 ist eine weitere Darstellung des zweiten Ausführungsbeispiels der Anordnung aus Fig. 4 von unten;
Fig. 6 ist eine Darstellung einer Befestigungsklammer gemäß einem dritten Ausführungsbeispiel der vorliegenden Offenbarung;
Fig. 7 ist eine seitliche Darstellung einer Anordnung mit dem dritten Ausführungsbeispiel der Befestigungsklammer aus Fig. 6;
Fig. 8 ist eine weitere Darstellung des dritten Ausführungsbeispiels der Anordnung aus Fig. 7 von unten.

### Beschreibung der Ausführungsbeispiele

Nachstehend werden drei Ausführungsbeispiele der vorliegenden Offenbarung auf der Basis der zugehörigen Figuren beschrieben.

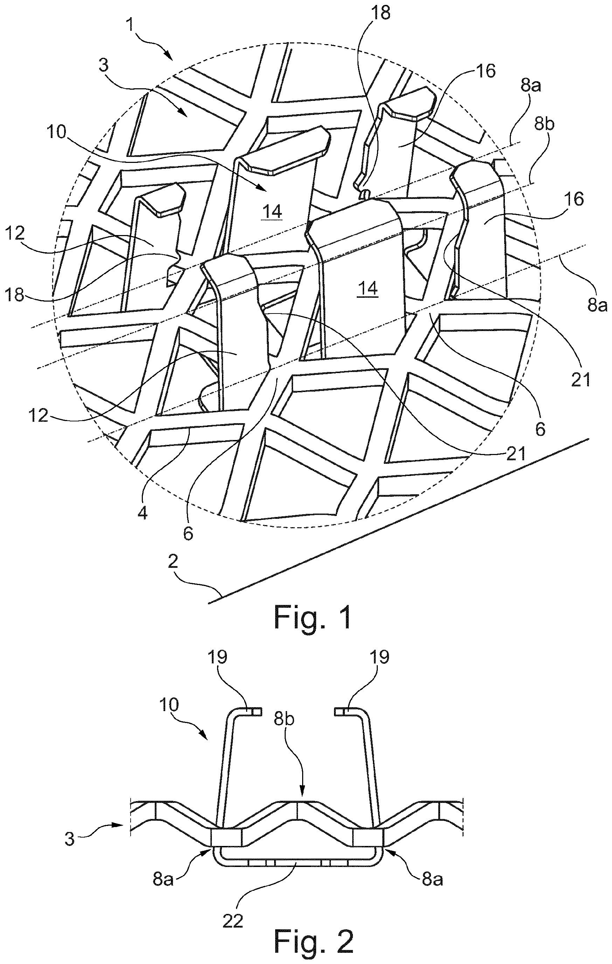

Fig. 1 zeigt einer Anordnung gemäß einem ersten Ausführungsbeispiel der vorliegenden Offenbarung. Sie hat einen Sterilisationssiebkorb 1, von dem nur einer seiner Ränder 2 und ein Ausschnitt seines Wellenbodens 3 gezeigt sind. Der Wellenboden 3 hat ein Perforationsgitter, das aus (in einer Draufsicht) rechtwinklig zueinander angeordneten Stegen 4 gebildet ist. Die Stege 4 sind bevorzugt unter 45° zum Rand 2 angestellt. Jeweils vier Stege 4 bilden ein Stegkreuz 6.

Parallel zum Rand 2 des Sterilisationssiebkorbes 1 erstrecken sich auf zwei verschiedenen Höhen Stegkreuzreihen 8a, 8b, wobei immer abwechselnd eine untere Stegkreuzreihe 8a und ein obere Stegkreuzreihe 8b vorgesehen sind. Demzufolge kann natürlich von dem genannten Winkel auch nach oben oder unten abgewichen werden, solange sich eine entsprechende Wellenform ergibt.

Durch die (in einer Draufsicht gesehen) rechtwinkligen Öffnungen des Perforationsgitters ist gemäß Fig. 1 eine größere Version der offenbarten Befestigungsklammer 10 gesteckt und verclipst. Genauer gesagt erstrecken sich parallel zu den Stegkreuzreihen 8a, 8b und parallel zum Rand 2 eine erste Armreihe und eine zweite Armreihe, wobei jede Armreihe einen ersten Haltearm 12, einen als Lasche ausgebildeten Zwischenarm 14 und eine zweiten Haltearm 16 hat. Die beiden Zwischenarme 14 sind so schmal ausgebildet, dass sie sich ohne direkte Berührung der Stege 4 oder der Stegkreuze 6 durch eine jeweilige Öffnung des Perforationsgitters erstrecken. Die vier Haltearme 12, 16 habe jeweils eine Rastnase 18, die jeweils an der Oberseite eines Stegkreuzes 6 anliegt. Damit ist die Befestigungsklammer 10 an den Wellenboden 3 des Sterilisationssiebkorbes 1 geclipst.

An den vier Haltearmen 12, 16 sind oberhalb der jeweiligen Rastnase 18 jeweilige Einführschrägen 21 gebildet. Damit ist für jedes der vier betroffenen Stegkreuze 6 eine Einführhilfe gebildet.

Fig. 2 ist eine weitere Darstellung des ersten Ausführungsbeispiels der Anordnung aus Figur 1 mit einer Blickrichtung entlang der Stegkreuzreihen 8a, 8b. An den oberen Endabschnitten der Haltearme 12, 16 und der Zwischenarme 14 sind Halteabschnitte 19 gebildet, die einen Silikonsteg des (nicht gezeigten) Instrumentenhalters umgreifen, der somit auch parallel zu den Stegkreuzreihen 8a, 8b angeordnet wird.

Gemäß Fig. 2 ist die Befestigungsklammer 10 an zwei unteren Stegkreuzreihen 8a angeclipst, so dass ein flächiger Hauptabschnitt 22 der Befestigungsklammer 10 unter dem Wellenboden 3 angeordnet ist. Auch diese so genannte untere Montageposition der Befestigungsklammer10 ist möglich.

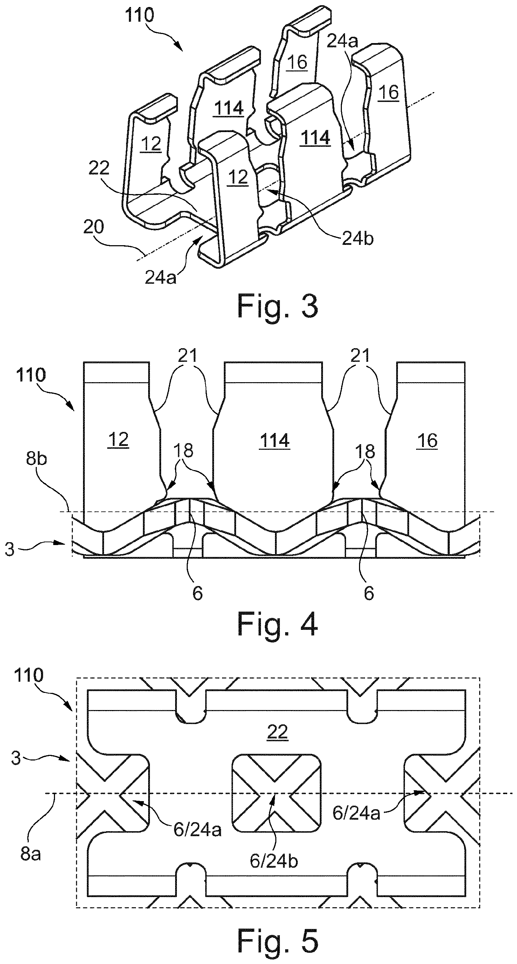

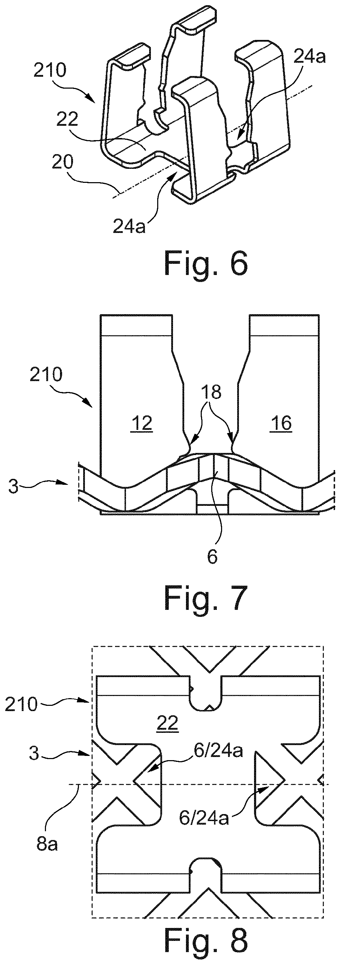

Wenn hingegen eine obere Montageposition gewählt wird, ist es möglich, dass der Hauptabschnitt 22 oberhalb der untere Stegkreuzreihe 8a angeordnet ist und so komplett in den Wellenboden 3 eintaucht. Figuren 4 und 7 zeigen Befestigungsklammern 110, 210 jeweils in der oberen Montageposition.

Fig. 3 ist eine Darstellung einer Befestigungsklammer 110 gemäß einem zweiten Ausführungsbeispiel der vorliegenden Offenbarung und gemäß der Erfindung.

Zunächst werden die Merkmale erläutert, die der Befestigungsklammer 10 aus Fig. 1 entsprechen: Die Befestigungsklammer 10, 110 ist ein Blechbiegeteil. Entlang einer Mittelachse 20, die eine Symmetrieachse ist, erstrecken sich zwei Armreihen mit jeweils einem ersten Haltearm 12, einem Zwischenarm 14, 114 und einem zweiten Haltearm 16. Zwischen den beiden Armreihen ist der Hauptabschnitt 22 angeordnet. In dem Hauptabschnitt 22 sind drei Ausnehmungen 24a, 24b vorgesehen, in die jeweils ein Stegkreuz 6 einer unteren Stegkreuzreihe 8a von oben eintauchen kann. Genauer gesagt sind zwei äußere Ausnehmungen 24a und eine als mittlere Durchgangsausnehmung gebildete weitere Ausnehmung 24b vorgesehen. Die beiden äußere Ausnehmungen 24a sind u-förmig und am Rand des Hauptabschnitts 22 angeordnet.

Im Unterschied zum ersten Ausführungsbeispiel der Befestigungskammer 10 aus Fig. 1 hat die zweite Befestigungsklammer 110 gemäß Figs. 3 bis 5 nicht nur an den Haltearmen 12, 16 Rastnasen 18, sondern auch an jedem Zwischenarm 114 beidseitig jeweils eine weitere Rastnase 18. Jede Rastnase 18 des Zwischenarms 114 erstreckt sich entgegen einer Rastnase 18 eines Haltearms 12, 16.

Fig. 4 ist eine seitliche Darstellung einer Anordnung mit dem Wellenboden 3 aus Fig. 1 und mit dem zweiten Ausführungsbeispiel der Befestigungsklammer 110 aus Fig. 3, in der die vier Rastnasen 18 einer Armreihe deutlich dargestellt sind. Somit kann jede Armreihe mit zwei Stegkreuzen 6 in Anlage kommen und das Stegkreuz 6 beidseitig halten.

An den vier Haltearmen 12, 16 und an den beiden Zwischenarmen 114 sind oberhalb der Rastnasen 18 jeweilige Einführschrägen 21 gebildet. Damit ist für jedes der vier betroffenen Stegkreuze 6 ein v-förmiger Einführtrichter gebildet.

Fig. 5 ist eine weitere Darstellung des zweiten Ausführungsbeispiels der Anordnung aus Fig. 4 von unten. Hier ist der Hauptabschnitt 22 komplett dargestellt, wobei in den drei Ausnehmungen 24a, 24 b jeweils ein Stegkreuz 6 der unteren Stegkreuzreihe 8a aufgenommen ist. Damit ist die obere Montageposition erzielt, bei der der Hauptabschnitt 22 die Abstellposition des Sterilisationssiebkorbes 1 nicht beeinflusst, auch wenn dieser fußlos ist.

Fig. 6 ist eine Darstellung einer Befestigungsklammer 210 gemäß einem dritten Ausführungsbeispiel der vorliegenden Offenbarung und gemäß der Erfindung. Es handelt sich um die so genannte kleinere Version, die entlang der Mittelachse 20 kürzer ist, als die beiden in den Figs. 1 bis 5 gezeigte größere Version. Beim dritten Ausführungsbeispiel sind die beiden Zwischenarme 14 entfallen.

Fig. 7 ist eine seitliche Darstellung einer Anordnung mit dem dritten Ausführungsbeispiel der Befestigungsklammer 210 aus Fig. 6. Es ist zu erkennen, dass die beiden Haltearme 12, 16 jeder Armreihe direkt zwischen sich nur ein Stegkreuz 6 aufnehmen.

Fig. 8 ist eine weitere Darstellung des dritten Ausführungsbeispiels der Anordnung aus Fig. 7 von unten. Beim dritten Ausführungsbeispiel ist die weitere Ausnehmung 24b am Hauptabschnitt 22 entfallen, da lediglich zwei Stegkreuze 6 der unteren Stegkreuzreihe 8a in eine jeweilige Ausnehmung 24a eintauchen müssen.

Die Figuren 4, 5, 7 und 8 zeigen die Befestigungsklammern 110; 210 in der bevorzugten höheren Montageposition am Wellenboden 3 des Sterilisationssiebkorbes 1, bei der die beiden Armreihen jeweils mit einer oberen Stegkreuzreihe 8b in Wirkverbindung sind, während die Ausnehmungen 24a, 24b Stegkreuze 6 einer unteren Stegkreuzreihe 8a aufnehmen.

Zusammenfassend ist in den vorstehenden Ausführungsbeispielen also eine Befestigungsklammer gezeigt, die zur werkzeuglosen Befestigung an dem perforierten bzw. gitterartigen Wellenboden 3 eines Sterilisationssiebkorbes ausgelegt ist. Hierfür hat die Befestigungsklammer zumindest die folgenden Merkmale:
- einen vorzugsweise platten- oder zungenförmigen Hauptabschnitt oder Grundplatte 22, an dem zwei bezüglich einer Mittel- oder Längsachse 20 des zungenförmigen (in Draufsicht im Wesentlichen rechteckigen) Hauptabschnitts einander gegenüberliegende Armreihen angeordnet bzw. ausgeformt sind. D.h. es sind mehrere vorzugsweise plattenförmige Arme auf jeder Längsseite des zungenförmigen Hauptabschnitts ausgeformt oder angeordnet, die in Längsrichtung des Hauptabschnitts jeweils voneinander beabstandet sind.
- Jede Armreihe weist zumindest einen ersten Haltearm 12 und einen zweiten Haltearm 16 auf, die sich im Wesentlichen senkrecht oder in einem Winkel um die 90° zum platten-/zungenförmigen Hauptabschnitt 22 erstrecken.
- An den einander zugewandten Seitenkanten von wenigstens zwei der Arme jeder Längsseite/Armreihe ist jeweils eine Rastnase oder Rastvorsprung 18 gebildet, die einander gegenüberliegen und auf einander zu weisen.
- An dem Hauptabschnitt 22 zwischen den beiden ersten Haltearmen 12 erstreckt sich ein erster Rand und zwischen den beiden zweiten Haltearmen 16 erstreckt sich ein zweiter Rand, wobei an den beiden Rändern jeweils eine Aussparung oder Ausnehmung 24a gebildet ist. In anderen Worten ausgedrückt sind an den längsbeabstandeten Stirnkanten des (platten-/zungenförmigen) Hauptabschnitts vorzugsweise rechteckige Ausnehmungen oder Einkerbungen 24a ausgeformt, die sich in Längsrichtung erstrecken. Die Ecken der vorzugsweise rechteckigen Ausnehmungen sind vorzugsweise abgerundet.

Das bedeutet, dass die Befestigungsklammer wenigstens die vier Klammerarme 12, 16 mit einander gegenüberliegender Rastnasen 18 hat, die an der gemeinsamen Basisplatte (Hauptabschnitt) 22 ausgeformt oder angeordnet sind und sich in einem Winkel um die 90° (plus/minus 20°) weg erstrecken, wobei immer wenigstens zwei Klammerarme an jeweils zwei gegenüberliegenden Längsseiten der Basisplatte 22 im Längsabstand zueinander angeordnet sind. In der Basisplatte 22 ist zumindest eine, vorzugsweise rechteckige, Ausnehmung 24a, 24b ausgeformt. Diese kann als eine rundum geschlossene, lochartige Ausnehmung 24b in einem Mittenbereich der Basisplatte 22 und/oder als eine teiloffene, kerbartige Ausnehmung 24a an wenigstens einer, die beiden Längsseiten verbindenden Stirnkante (Querseite) oder an beiden Stirnkanten (Querseiten) der Basisplatte 22 ausgebildet sein.

### Bezugszeichenliste:

- 1: Sterilisationssiebkorb
- 2: Rand des Sterilisationssiebkorbes
- 3: Wellenboden
- 4: Steg
- 6: Stegkreuz
- 8a: untere Stegkreuzreihe
- 8b: obere Stegkreuzreihe
- 10: größere Version der Befestigungsklammer (erste Variante)
- 12: erster Haltearm
- 14: Zwischenarm
- 16: zweiter Haltearm
- 18: Rastnase
- 19: Halteabschnitt
- 20: Mittelachse
- 21: Einführschräge
- 22: Hauptabschnitt
- 24a: Ausnehmung
- 24b: weitere Ausnehmung
- 110: größere Version der Befestigungsklammer (zweite Variante)
- 114: Zwischenarm
- 210: kleinere Version der Befestigungsklammer

## Patentansprüche

1. Befestigungsklammer, die zur werkzeuglosen Befestigung an einem perforierten Wellenboden (3) eines Sterilisationssiebkorbes (1) eingerichtet und ausgelegt ist, wobei die Befestigungsklammer einen Hauptabschnitt (22) hat, an dem zwei bezüglich einer Mittelachse (20) einander gegenüberliegende Armreihen angeordnet sind, wobei jede Armreihe einen ersten Haltearm (12) und einen zweiten Haltearm (16) hat, an denen jeweils eine Rastnase (18) gebildet ist, wobei die Rastnasen (18) der beiden Haltearme (12, 16) jeder Armreihe auf einander zu weisen, und wobei sich an dem Hauptabschnitt (22) zwischen den beiden ersten Haltearmen (12) ein erster Rand und zwischen den beiden zweiten Haltearmen (16) ein zweiter Rand erstreckt, **dadurch gekennzeichnet, dass** an den beiden Rändern jeweils eine Aussparung oder Ausnehmung (24a) gebildet ist.

2. Befestigungsklammer nach Anspruch 1, wobei sich die beiden Aussparungen oder Ausnehmungen (24a) quer zur Mittelachse (20) über mindestens ein Drittel einer jeweiligen Breite des Hauptabschnitts (22) oder eines jeweiligen Abstandes der einander gegenüberliegenden Haltearme (12, 16) erstrecken.

3. Befestigungsklammer nach einem der vorhergehenden Ansprüche, wobei sich die beiden Aussparungen oder Ausnehmungen (24a) entlang der Mittelachse (20) jeweils über mindestens ein Achtel einer Länge des Hauptabschnitts (22) erstrecken.

4. Befestigungsklammer nach einem der vorhergehenden Ansprüche, wobei zwischen den beiden Haltearmen (12, 16) einer Armreihe jeweils ein Zwischenarm (14; 114) vorgesehen ist, und wobei etwa mittig in dem Hauptabschnitt (20) eine weitere Ausnehmung (24b) vorgesehen ist, die als Durchgangsausnehmung ausgebildet ist.

5. Befestigungsklammer nach Anspruch 4, wobei an freien Endabschnitten der Zwischenarme (14; 114) und/oder ersten Haltearme (12) und/oder zweiten Haltearme (16) Halteabschnitte (19) gebildet sind, bevorzugt mindestens 3 Halteabschnitte (19).

6. Befestigungsklammer nach Anspruch 5, wobei die Halteabschnitte (19) einander paarweise gegenüberliegend, bevorzugt einander paarweise zugewandt, vorgesehen sind.

7. Befestigungsklammer nach einem der Ansprüche 4 bis 6, wobei an den beiden Zwischenarmen (114) jeweils eine weitere Rastnase (18) vorgesehen ist, die sich entgegen der Rastnase (18) des ersten Haltearms (12) erstreckt.

8. Befestigungsklammer nach einem der Ansprüche 4 bis 6, wobei der Zwischenarm (14) eine Lasche ist, an der gegenüber den Rastnasen (18) der beiden benachbarten Haltearme (12, 16) jeweils ein gerader Randabschnitt gebildet ist.

9. Befestigungsklammer nach einem der Ansprüche 4 bis 8, wobei die Durchgangsausnehmung im Wesentlichen rechteckig ist.

10. Befestigungsklammer nach einem der vorhergehenden Ansprüche, wobei oberhalb der Rastnasen (18) Einführschrägen (21) gebildet sind.

11. Anordnung mit mindestens einer Befestigungsklammer (10;110; 210) gemäß einem der vorhergehenden Ansprüche und mit einem Sterilisationssiebkorb (1), wobei ein Wellenboden (3) des Sterilisationssiebkorbes (1) ein Perforationsgitter aufweist, wobei jeweils vier Stege (4) des Perforationsgitters Stegkreuze (6) bilden, wobei die Stegkreuze (6) auf zwei unterschiedlichen Höhen des Wellenbodens (3) angeordnet sind, wobei an dem Wellenboden (3) die mindestens eine Befestigungsklammer (10; 110; 210) anclipsbar oder angeclipst ist.

12. Anordnung nach Anspruch 11, wobei die Stegkreuze (6) in Stegkreuzreihen (8a, 8b) angeordnet sind, die abwechselnd auf den beiden unterschiedlichen Höhen des Wellenbodens (3) angeordnet sind, wobei sich die Stegkreuzreihen (8a, 8b) entlang dem gesamten Wellenboden (3) und parallel zu einem Rand (2) des Wellenbodens (3) oder des Sterilisationssiebkorbes (1) erstrecken.

13. Anordnung nach Anspruch 12, wobei zwischen den beiden Haltearmen (12, 16) jeder Armreihe der mindestens einen Befestigungsklammer (10;110; 210) jeweils ein oder zwei Stegkreuze (6) einer Stegkreuzreihe (8a, 8b) angeordnet ist oder sind, und wobei das Stegkreuz (6) oder die beiden Stegkreuze (6) an den Rastnasen (18) anliegen.

14. Anordnung nach Anspruch 13, wobei zwei oder drei zueinander benachbarte Stegkreuze (6) einer unteren Stegkreuzreihe (8b) jeweils in eine Ausnehmung (24a, 24b) des Hauptabschnitts (22) eintauchen.

15. Anordnung nach einem der Ansprüche 11 bis 14, wobei die Stege (4) des Wellenbodens (3) um 45° zu einem Rand (2) des Wellenbodens (3) oder des Sterilisationssiebkorbes (1) angestellt sind, und wobei die jeweilige Mittelachse (20) der mindestens einen Befestigungsklammer (10; 110; 210) etwa parallel zum Rand (2) des Wellenbodens (3) oder des Sterilisationssiebkorbes (1) angeordnet ist.

## Claims

1. A fastening clamp that is adapted and configured for attachment without tools to a perforated undulating base (3) of a sterilization sieve basket (1), wherein the fastening clamp has a main portion (22) on which two rows of arms are arranged opposite each other with respect to a central axis (20), wherein each row of arms has a first retaining arm (12) and a second retaining arm (16), on each of which a respective latching nose (18) is formed, wherein the latching noses (18) of the two retaining arms (12, 16) of each row of arms face toward each other, and wherein a first edge extends on the main portion (22) between the two first retaining arms (12) and a second edge extends between the two second retaining arms (16), **characterized in that** a cutout or recess (24a) is formed on each of the two edges.

2. The fastening clamp according to claim 1, wherein the two cutouts or recesses (24a) extend transversely to the central axis (20) over at least one third of a respective width of the main portion (22) or of a respective distance of the opposite retaining arms (12, 16).

3. The fastening clamp according to one of the preceding claims, wherein the two cutouts or recesses (24a) along the central axis (20) each extend over at least one-eighth of a length of the main portion (22).

4. The fastening clamp according to one of the preceding claims, wherein a respective intermediate arm (14; 114) is provided between the two retaining arms (12, 16) of a row of arms, and wherein a further recess (24b) is provided approximately in the center of the main portion (20), which is configured as a passage recess.

5. The fastening clamp according to claim 4, wherein holding portions (19), preferably at least three holding portions (19), are formed on free end portions of the intermediate arms (14; 114) and/or on first retaining arms (12) and/or on second retaining arms (16).

6. The fastening clamp according to claim 5, wherein the holding portions (19) are provided opposite each other in pairs, preferably facing each other in pairs.

7. The fastening clamp according to one of claims 4 to 6, wherein a further latching nose (18) is provided on each of the two intermediate arms (114), which extends counter to the latching nose (18) of the first retaining arm (12).

8. The fastening clamp according to one of claims 4 to 6, wherein the intermediate arm (14) is a tab on which a respective straight edge portion is formed opposite the latching noses (18) of the two adjacent retaining arms (12, 16).

9. The fastening clamp according to one of claims 4 to 8, wherein the passage recess is substantially rectangular.

10. The fastening clamp according to one of the preceding claims, wherein insertion chamfers (21) are formed above the latching noses (18).

11. An arrangement comprising at least one fastening clamp (10; 110; 210) according to one of the preceding claims and a sterilization sieve basket (1), wherein an undulating base (3) of the sterilization sieve basket (1) comprises a perforated grid, wherein respectively four crosspieces (4) of the perforated grid form crosspiece junctions (6), wherein the crosspiece junctions (6) are arranged at two different heights of the undulating base (3), wherein the at least one fastening clamp (10; 110; 210) is clippable or clipped to the undulating base (3).

12. The arrangement according to claim 11, wherein the crosspiece junctions (6) are arranged in crosspiece junction rows (8a, 8b) which are alternately arranged on the two different heights of the undulating base (3), wherein the crosspiece junction rows (8a, 8b) extend along the entire undulating base (3) and parallel to an edge (2) of the undulating base (3) or of the sterilization sieve basket (1).

13. The arrangement according to claim 12, wherein in each case one or two between the two retaining arms (12, 16) of each row of arms, and wherein the crosspiece junction (6) or the two crosspiece junctions (6) abut on the latching noses (18).

14. The arrangement according to claim 13, wherein two or three crosspiece junctions (6) of a lower crosspiece junction row (8b) adjacent to each other each dip into a recess (24a, 24b) of the main portion (22).

15. The arrangement according to one of claims 11 to 14, wherein the crosspieces (4) of the undulating base (3) are angled at 45° to an edge (2) of the undulating base (3) or of the sterilization sieve basket (1), and wherein the respective central axis (20) of the at least one fastening clamp (10; 110; 210) is arranged approximately parallel to the edge (2) of the undulating base (3) or of the sterilization sieve basket (1).

## Revendications

1. Pince de fixation qui est conçue et configurée pour la fixation sans outil à une base ondulée perforée (3) d'un panier maillé de stérilisation (1), dans laquelle la pince de fixation a une partie principale (22) au niveau de laquelle sont agencées deux séries de bras en regard l'une de l'autre par rapport à un axe central (20), dans laquelle chaque série de bras a un premier bras de retenue (12) et un second bras de retenue (16) au niveau desquels est respectivement formée un nez d'encliquetage (18), dans laquelle les nez d'encliquetage (18) des deux bras de retenue (12, 16) de chaque série de bras sont orientées l'une vers l'autre, et dans laquelle un premier bord s'étend entre les deux premiers bras de retenue (12) et un second bord s'étend entre les deux seconds bras de retenue (16) au niveau de la partie principale (22), **caractérisée en ce qu'**au niveau des deux bords un renfoncement ou un évidement (24a) est respectivement formé.

2. Pince de fixation selon la revendication 1, dans laquelle les deux renfoncements ou évidements (24a) s'étendent transversalement à l'axe central (20) sur au moins un tiers d'une largeur respective de la partie principale (22) ou d'un écart respectif des bras de retenue (12, 16) qui se font face.

3. Pince de fixation selon l'une quelconque des revendications précédentes, dans laquelle les deux renfoncements ou évidements (24a) s'étendent le long de l'axe central (20) respectivement sur un huitième d'une longueur de la partie principale (22).

4. Pince de fixation selon l'une quelconque des revendications précédentes, dans laquelle respectivement un bras intermédiaire (14 ; 114) est prévu entre les deux bras de retenue (12, 16) d'une série de bras, et dans laquelle est prévu un autre évidement (24b) qui est réalisé en tant qu'évidement de passage à peu près au milieu de la partie principale (20).

5. Pince de fixation selon la revendication 4, dans laquelle des sections de retenue (19) sont formées, de préférence au moins 3 sections de retenue (19), au niveau de parties d'extrémité libres des bras intermédiaires (14 ; 114) et/ou des premiers bras de retenue (12) et/ou des seconds bras de retenue (16).

6. Pince de fixation selon la revendication 5, dans laquelle les sections de retenue (19) sont prévues opposées par paires les unes aux autres, de préférence orientées par paire les unes vers les autres.

7. Pince de fixation selon l'une quelconque des revendications 4 à 6, dans laquelle un autre nez d'encliquetage (18) est respectivement prévu au niveau des deux bras intermédiaires (114), qui s'étend dans le sens opposé du nez d'encliquetage (18) du premier bras de retenue (12).

8. Pince de fixation selon l'une quelconque des revendications 4 à 6, dans laquelle le bras intermédiaire (14) est une languette au niveau de laquelle une partie de bord droite est respectivement formée de manière opposée aux nez d'encliquetage (18) des deux bras de retenue (12, 16) voisins.

9. Pince de fixation selon l'une quelconque des revendications 4 à 8, dans laquelle l'évidement de passage est sensiblement rectangulaire.

10. Pince de fixation selon l'une quelconque des revendications précédentes, dans laquelle des pentes d'introduction (21) sont formées au-dessus des nez d'encliquetage (18).

11. Agencement avec au moins une pince de fixation (10; 110; 210) selon l'une quelconque des revendications précédentes et avec un panier maillé de stérilisation (1), dans lequel une base ondulée (3) du panier maillé de stérilisation (1) présente une grille de perforation, dans lequel respectivement quatre entretoises (4) de la grille de perforation forment des croix d'entretoise (6), dans lequel les croix d'entretoise (6) sont disposées sur deux hauteurs différentes de la base ondulée (3), dans lequel la au moins une pince de fixation (10 ; 110 ; 210) est clipsable ou clipsée au niveau de la base ondulée (3).

12. Agencement selon la revendication 11, dans lequel les croix d'entretoise (6) sont disposées en séries de croix d'entretoise (8a, 8b) qui sont disposées en alternance sur les deux hauteurs différentes de la base ondulée (3), dans lequel les séries de croix d'entretoise (8a, 8b) s'étendent le long de l'ensemble de la base ondulée (3) et parallèlement à un bord (2) de la base ondulée (3) ou du panier maillé de stérilisation (1).

13. Agencement selon la revendication 12, dans lequel respectivement une ou deux croix d'entretoise (6) d'une série de croix d'entretoise (8a, 8b) est/sont disposée(s) entre les deux bras de retenue (12, 16) de chaque série de bras de la au moins une pince de fixation (10; 110; 210), et dans lequel la croix d'entretoise (6) ou les deux croix d'entretoise (6) sont adjacentes aux nez d'encliquetage (18).

14. Agencement selon la revendication 13, dans lequel deux ou trois croix d'entretoise (6) voisines les unes par rapport aux autres d'une série de croix d'entretoise inférieure (8b) plongent respectivement dans un évidement (24a, 24b) de la partie principale (22).

15. Agencement selon l'une quelconque des revendications 11 à 14, dans lequel les entretoises (4) de la base ondulée (3) sont réglées à 45° par rapport à un bord (2) de la base ondulée (3) ou du panier maillé de stérilisation (1), et dans lequel l'axe central respectif (20) de la au moins une pince de fixation (10 ; 110 ; 210) est disposé à peu près parallèlement à un bord (2) de la base ondulée (3) ou du panier maillé de stérilisation (1).
